# EUROPÄISCHE PATENTANMELDUNG

(11) **EP 1 881 129 A2**
(43) Veröffentlichungstag der Anmeldung: **23.01.2008**
(21) Anmeldenummer: 07112742.7
(22) Anmeldetag: 19.07.2007
(51) Int. Cl.: E04G 21/32, E04G 3/28, F17B 1/00

(54) **Behälterartiges Bauwerk mit einer Sicherungseinrichtung**

(30) Priorität: 20.07.2006 DE 102006034096; 21.02.2007 DE 202007002711 U
(71) Anmelder: MT-Energie GmbH & Co. KG, 27404 Rockstedt (DE)
(72) Erfinder: Martens, Christoph, 27404, Rockstedt (DE)
(74) Vertreter: von Ahsen, Erwin-Detlef

(57) **Zusammenfassung**

Die Erfindung betrifft ein behälterartiges Bauwerk mit einer umlaufenden Wand (11), welche einen nach oben offenen Innenraum umschließt. Um Montage- und Wartungsarbeiten an dem Bauwerk schnell und damit kostengünstig durchführen zu können, ist das erfindungsgemäße Bauwerk dadurch gekennzeichnet, daß der Innenraum mittels einer Folie (31, 32) abgedeckt ist, wobei an der Folie (31, 32) ein Befestigungsmittel (33) für eine Sicherheitseinrichtung angeordnet ist.

## Beschreibung

Die Erfindung betrifft ein behälterartiges Bauwerk, insbesondere einen Gärbehälter für eine Biogasanlage, mit einer umlaufenden Wand, welche einen nach oben offenen Innenraum umschließt.

Derartige Bauwerke sind aus der GB 270 893 bekannt.

Solche Bauwerke werden zum Beispiel als Gärbehälter (Fermenter) von Biogasanlagen verwendet. Diese Bauwerke weisen eine einen geschlossenen Kreis bildende umlaufende Wand auf. An der oberen Kante der Wand stützt sich ein Dach ab. Häufig werden Dächer aus Dachspanten verwendet, welche mit einer Folie abgedeckt sind. Diese Folie bildet das eigentliche Dach des Bauwerks. Bei Biogasfermentern werden meist Tragluftfoliendächer verwendet.

Um an nach oben offenen, behälterartigen Bauwerken, wie beispielsweise Biogasfermentern, Arbeiten durchführen zu können, wie zum Beispiel das Anbringen einer Abdeckung oder sonstige Montage- oder Wartungsarbeiten, müssen Personen zum Teil in erhöhten Ebenen arbeiten. Um diese Personen gegen Absturz zu sichern, müssen entsprechende Sicherungseinrichtungen vorgesehen werden. Dieses erfolgt in der Praxis durch Anbringen eines entsprechenden Gurtes, welcher an einer zuvor an der Wand befestigten Öse eingehängt wird. Dabei muß der Gurt im Laufe der Arbeiten mehrfach umgehängt werden, was aufwendig ist. Auch Leitern, auf denen die Personen meist arbeiten, müssen umgesetzt werden.

Hiervon ausgehend liegt der Erfindung das Problem zugrunde, ein behälterartiges Bauwerk der eingangs genannten Art derart weiterzubilden, daß Montage- und Wartungsarbeiten an dem Bauwerk schnell und damit kostengünstig ausführbar sind.

Zur Lösung dieses Problems ist das erfindungsgemäße behälterartige Bauwerk dadurch gekennzeichnet, daß der Innenraum mittels einer Folie abgedeckt ist, wobei an der Folie ein Befestigungsmittel für eine Sicherheitseinrichtung angeordnet ist.

Wie bereits erwähnt, werden Biogasfermenter mit einem Dach aus einer Tragluftfolie abgedeckt. Unterhalb der Tragluftfolie ist eine weitere (Membran-)Folie zur Abdeckung des Innenraums des Fermenters eingezogen, welche den eigentlichen Speicherraum für das Biogas bildet. Es hat sich gezeigt, daß das bloße Auflegen der Folie auf die Tragbalken bereits hinreichende Reibungskräfte verursacht, so daß die Folie selbst beim Anbringen einer Sicherheitseinrichtung an der Folie nicht verrutscht und so eine eingehängte Person ausreichend sichert. Dies hat den Vorteil, daß keine gesonderten Bauteile erforderlich sind. Vielmehr ist die Anbringung des Sicherungsmittels direkt an der ohnehin erforderlichen Folie möglich. Vorteilhafterweise ist das Befestigungsmittel etwa mittig an der Folie angeordnet.

Nach einer weiteren Ausführungsform ist im Bereich des oberen Randes der Wand eine umlaufende Schiene oder ein Seil angeordnet. An dieser umlaufenden Schiene lassen sich Sicherungseinrichtungen für die Personen und/oder Arbeitsgerüste anbringen. Die Sicherungseinrichtung oder das Arbeitsgerüst sind an der Schiene verfahrbar und können so auf einfache Weise ohne große Umhängarbeiten an dem Bauwerk umgesetzt werden.

Die Schiene ist im Querschnitt vorzugsweise L-, C-, U- oder T-förmig oder als Flachstahl ausgebildet mit wenigstens einem etwa parallel zur Wand verlaufenden Steg. Hierdurch läßt sich die Schiene durch entsprechende Einrichtungen an der Sicherungseinrichtung oder dem Arbeitsgerüst so sichern, daß auch von der Wand weg gerichtete Kräfte in einer senkrecht zur Wand verlaufenden Richtung aufgenommen werden können. Dieses ist insbesondere dann von Vorteil, wenn an der Schiene eine Sicherungseinrichtung oder ein Arbeitsgerüst an der von der Schiene abgewandten Wandseite angebracht werden muß.

Nach einer weiteren Ausführungsform weist die Schiene zwei keilförmig aufeinander zu verlaufende Füße auf, an deren von der Wand abgewandten Ende der Steg angebracht ist. Auf diese Weise ist die Schiene auch in einer Richtung parallel zur Wand versteift, was der Stabilität dient.

Die Schiene dient vorzugsweise zum Anbringen einer Sicherungseinrichtung. In diese Sicherungseinrichtung können sich auf dem Bauwerk bzw. an dem Bauwerk arbeitende Personen einhängen und gegen Absturz sichern. Durch die Anordnung der Sicherungseinrichtung an der Schiene ist das Bauwerk rundherum für die Personen zugänglich, ohne daß sie sich umhängen müssen. Zusätzlich oder alternativ kann an der Schiene auch ein Arbeitsgerüst angeordnet sein. Ergänzt werden kann die Sicherheitseinrichtung und/oder das Arbeitsgerüst durch eine weitere Sicherungseinrichtung, welche insbesondere ein aufrechter, etwa mittig im Bauwerk angeordneter Stab sein kann. Dieser ist mit der zweiten Sicherungseinrichtung durch eine Laufleine oder eine Stange verbunden. Die zweite Sicherungseinrichtung bzw. das Arbeitsgerüst können also um das Bauwerk herum entlang der Schiene verfahren werden. Gleichzeitig ist etwa mittig im Bauwerk die weitere Sicherungseinrichtung angeordnet, die durch eine Laufleine oder durch eine Stange mit der zweiten verbunden ist. Hierdurch ist eine größtmögliche Bewegungsfreiheit auf der Oberseite des Bauwerks gegeben. Alternativ können auch zwei einander diametral gegenüberliegende Sicherungseinrichtungen an der Schiene vorgesehen sein, die durch eine Laufleine oder eine Stange miteinander verbunden sind. Auch hier ergibt sich eine größtmögliche Bewegungsfreiheit auf der Oberseite des Bauwerks.

Nach einer weiteren Ausführungsfom, die auch unabhängig von der vorliegenden Erfindung denkbar ist, weist das Befestigungsmittel für Sicherheitseinrichtungen an Foliendächern, insbesondere für Biogasfermenter, ein Befestigungsorgan und eine Öse für die Sicherheitseinrichtung auf und das Befestigungsorgan ist zangenartig ausgebildet und an dem Foliendach festklemmbar.

Biogasfermenter werden mit unterschiedlichen Arten von Dächern versehen. Häufig verbreitet sind Foliendächer, die entweder zirkuszeltartig mit einer inneren Stütze im Zentrum des Fermenters abgestützt werden, oder sogenannte Tragluftfoliendächer, bei welchen das Foliendach aufgrund eines inneren Überdrucks im Fermenter getragen wird. Beim Erstellen derartiger Foliendächer klettern die Monteure auf dem Fermenter und gegebenenfalls vorhandenen Spannstreben für das Foliendach herum. Dabei besteht Absturzgefahr, so daß sich die Monteure gegen Absturz sichern müssen. Hierzu laschen sie sich mittels Sorgleinen an geeigneten Einrichtungen fest. Die Anbringung der Sorgleinen war bisher aber oft schwierig und auch für die Monteure unbequem, so daß entgegen aller Vernunft auf die Sorgleinen verzichtet wurde.

Aufgrund der vorstehend genannten Ausführungsform des Befestigungsmittels kann auf vorteilhafte Weise eine einfache und effiziente Anbringung für Sicherheitseinrichtungen, insbesondere für die Sorgleine, bereitgestellt werden.

Beim Erstellen der Foliendächer werden die Folien zunächst grob auf dem Fermenter und gegebenenfalls vorhandenen Stützstreben ausgebreitet. Aufgrund der Konstruktion des Befestigungsmittels läßt sich dieses etwa zentrisch an der grob ausgebreiteten Folie festklemmen. Dabei liegt dem auch unabhängig denkbaren Befestigungsmittel die Erkenntnis zugrunde, daß zwischen dem Foliendach und dem Fermenter bzw. eventuell vorhandenen Stützstreben bereits eine ausreichende Reibung vorhanden ist, daß eine eventuell abstürzende Person sicher aufgefangen wird. Das Befestigungsmittel läßt sich auf einfache Weise schnell an der Folie anbringen und ist für Monteure bequem zu handhaben. Die Sorgleine kann ohne weiteres an der Öse befestigt werden.

Nach einer Weiterbildung des Befestigungsmittels ist ein Abschnitt der Öse gleichzeitig auch ein Gelenk des zangenartigen Befestigungsorgans. Hierdurch wird in Verbindung mit der Reibung zwischen dem zangenartigen Befestigungsmittel und der Folie durch Zug an der Öse und damit an dem Gelenk bewirkt, daß das zangenartige Befestigungsorgan sich weiter schließt und hierdurch die Haltekräfte an der Folie noch erhöht bzw. auch bei nicht ausreichend geschlossenem Befestigungsorgan noch eine ausreichende Haltekraft vorhanden ist. Auf konstruktiv besonders einfache Weise läßt sich dieses erstellen, indem als Öse ein handelsüblicher Schäkel verwendet wird, dessen Sperrbolzen gleichzeitig das Gelenk bildet.

Das zangenartige Befestigungsorgan selbst sollte mittels einer Spannschraube feststellbar sein. Hierdurch läßt sich die Folie besonders einfach klemmen.

Die Erfindung wird nachfolgend anhand eines in der Zeichnung dargestellten Ausführungsbeispiels aufgrund mehrerer Anwendungsvarianten näher erläutert. In der Zeichnung zeigen:
Fig. 1 ein Bauwerk in Seitenansicht, das jedoch nicht in den Schutzbereich, wie durch den Hauptanspruch definiert, fällt,
Fig. 2 einen Abschnitt, nämlich den oberen Bereich einer Wand des Bauwerks gemäß Fig. 1,
Fig. 3 einen zweiten Anwendungsfall für ein Bauwerk gemäß Fig. 1,
Fig. 4 einen dritten Anwendungsfall für ein Bauwerk gemäß Fig. 1,
Fig. 5 einen vierten Anwendungsfall für ein Bauwerk gemäß Fig. 1,
Fig. 6 einen fünften Anwendungsfall für ein Bauwerk gemäß Fig. 1 in Draufsicht,
Fig. 7 den Anwendungsfall gemäß Fig. 6 in Vertikalschnitt,
Fig. 8 einen weiteren Anwendungsfall für ein Bauwerk gemäß Fig. 1 im Vertikalschnitt,
Fig. 9 ein Befestigungsmittel in perspektivischer Unteransicht und
Fig. 10 das Befestigungsmittel gemäß Fig. 9 in perspektivischer Draufsicht.

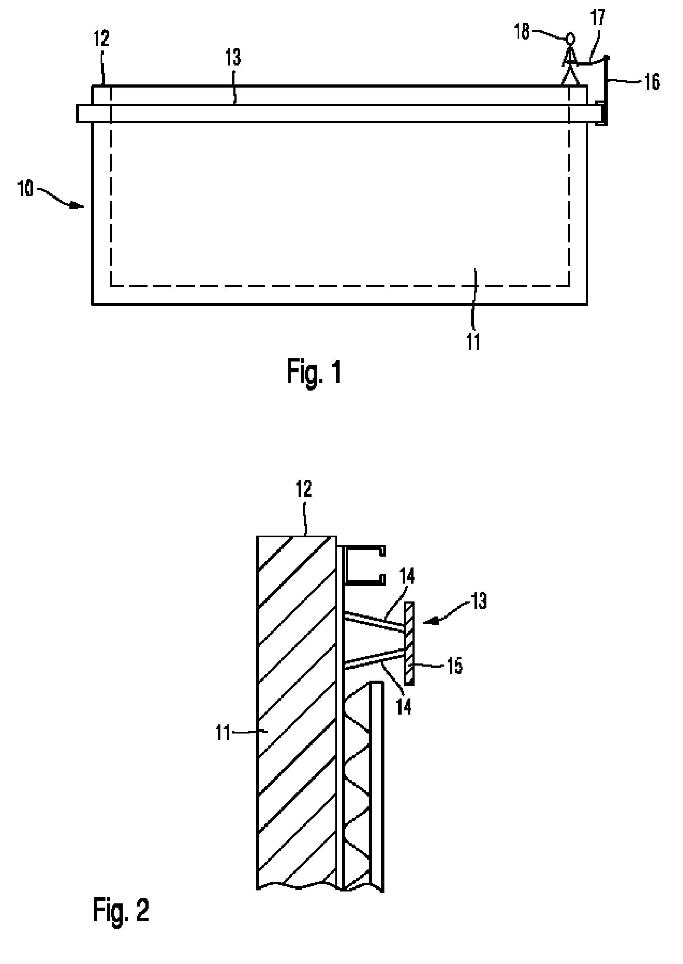

Das in Fig. 1 dargestellte Bauwerk, nämlich ein Biogasfermenter 10, weist eine auf einem Kreis vollständig umlaufenden, geschlossenen aufrechten Wand 11 auf. Knapp unterhalt des oberen Randes 12 der Wand 11 ist eine in Umfangsrichtung der Wand 11 vollständig umlaufende Schiene 13 angeordnet. Im vorliegenden Fall ist die Schiene 13 im Querschnitt etwa T-förmig ausgebildet mit zwei von der Wand 11 weg gerichteten Füßen 14, auf deren freies Ende ein in Umfangsrichtung der Wand 11 umlaufender Steg 15 angeschweißt ist. Der Steg 15 steht zu beiden Seiten gegenüber den Füßen 14 über und erstreckt sich parallel zur Wand 11. Die Füße 14 sind, wie in Fig. 2 erkennbar, so ausgebildet, daß sie in einer von der Wand 11 weg gerichteten Richtung keilförmig aufeinander zu verlaufen, also einen keilförmig verjüngenden Querschnitt bilden.

Fig. 1 zeigt bereits ein erstes Anwendungsbeispiel. An der Schiene 13 ist eine Sicherungseinrichtung 16 in Form einer aufrechten Stütze angeordnet. Diese umgreift, wie in Fig. 1 erkennbar, beide überstehenden Enden des Steges 15 C-artig. Am oberen, freien Ende der Sicherungseinrichtung 16 ist ein Sicherungsgurt 17 für eine auf dem oberen Rand 12 arbeitende Person 18 angebracht. Die Person 18 ist so gegen Herunterfallen gesichert. Sie kann auf dem oberen Rand 12 frei arbeiten und sich auf diesem entlang bewegen. Dabei verfährt die Sicherungseinrichtung 16 an der Schiene 13, so daß die Person 18 den Gurt 17 nicht umzuhängen braucht. An der Sicherungseinrichtung 16 können entsprechende Rollenlager vorgesehen sein, um ein Verfahren der Sicherungseinrichtung 16 an der Schiene 13 zu erleichtern.

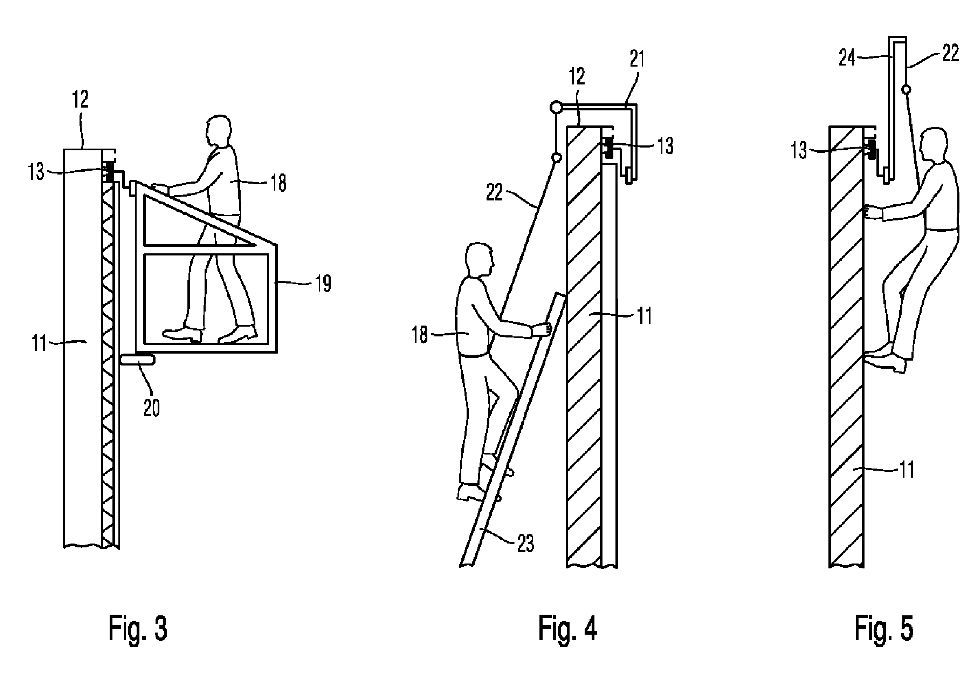

Fig. 3 zeigt einen Anwendungsfall, bei dem an der Schiene 13 ein Arbeitsgerüst 19 eingehängt ist. Konkret ist das Arbeitsgerüst 19 mit seinem oberen Ende an der Schiene 13 angebracht und stützt sich mit Rollen 20 an seinem unteren Rand an der Außenseite der Wand 11 ab. Das Arbeitsgerüst 19 ist ebenfalls mit entsprechenden Rollen an der Schiene 13 befestigt. Es läßt sich so an der Schiene 13 und an der Wand 11 entlang verfahren, ohne daß es umgehängt werden muß.

In Fig. 4 ist ein Anwendungsfall gezeigt, bei dem eine den oberen Rand 12 der Wand 11 galgenartig umgreifende Sicherungseinrichtung 21 eingehängt ist. Am freien Ende der Sicherungseinrichtung 21 ist wiederum ein Sicherungsgurt 22 eingehängt, der eine auf einer Leiter 23 stehende Person 18 sichert. Aufgrund der galgenartigen Ausbildung der Sicherungseinrichtung 21 kann die Person 18 an der Innenseite der Wand 11 arbeiten, obwohl die Schiene 13 an der Außenseite der Wand 11 angebracht ist.

Bei dem in Fig. 5 gezeigten Anwendungsbeispiel ist eine aufrechte Sicherungseinrichtung 24 angebracht, an deren oberen Ende wiederum der Sicherungsgurt 22 zur Sicherung einer Person 18 angebracht ist. Die Person 18 stützt sich hier mit ihren Füßen direkt an der Wand 11 ab und kann aufgrund der Verfahrmöglichkeit der Sicherungseinrichtung 24 an der Schiene 13 sich umlaufend an der Wand 11 fortbewegen.

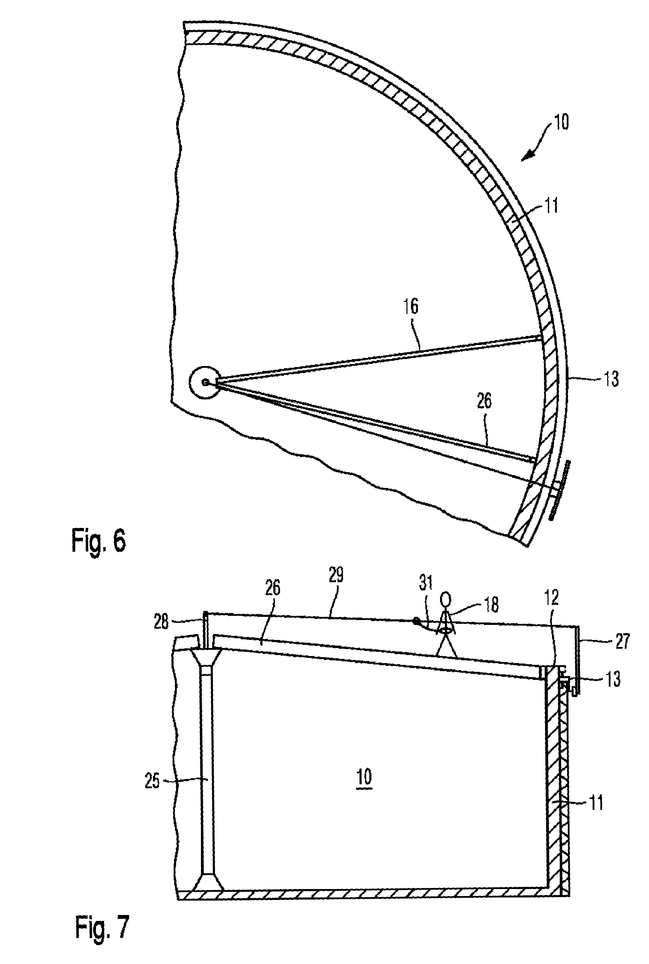

In den Fig. 6 und 7 ist ein Anwendungsfall gezeigt, bei dem ein Tragluftfoliendach auf dem Bauwerk, nämlich dem Biogasfermenter 10, angebracht wird. Etwa im Mittelpunkt des Behälters 10 ist eine aufrechte Stütze 25 angeordnet. Von der Stütze 25 weg verlaufen sternartig Balken 26, die sich einerseits an der Stütze 25 und andererseits am oberen Rand 12 der Wand 11 abstützen. Auf diesen Balken 26 wird später eine Folie gelegt und umlaufend an der Wand 11 gebildet. Diese Folie bildet später das Tragluftfoliendach.

Um diese und andere Arbeiten ausführen zu können, ist in der Schiene 13 wiederum eine Sicherungseinrichtung 27 analog der Sicherungseinrichtung 16 angebracht. Auf der Stütze 25 befindet sich ein aufrechter Stab 28. Die oberen Enden des Stabes 28 und der Sicherungseinrichtung 27 sind mit einer Laufleine 29, einer Stange oder dergleichen verbunden. An dieser Laufleine 29 oder dergleichen wird der Sicherungsgurt 30 verschieblich geführt. Die Person 18 kann so auf den Balken 26 gesichert entlanggehen sowie auch auf dem oberen Rand 12 der Wand 11. Insgesamt kann sich die Person 18 frei auf dem Biogasfermenter 10 bewegen.

Alternativ können an der Schiene 13 auch zwei einander etwa diametral gegenüberliegende Sicherungseinrichtungen 27 angeordnet sein, welche durch die Laufleine 29 oder dergleichen miteinander verbunden sind.

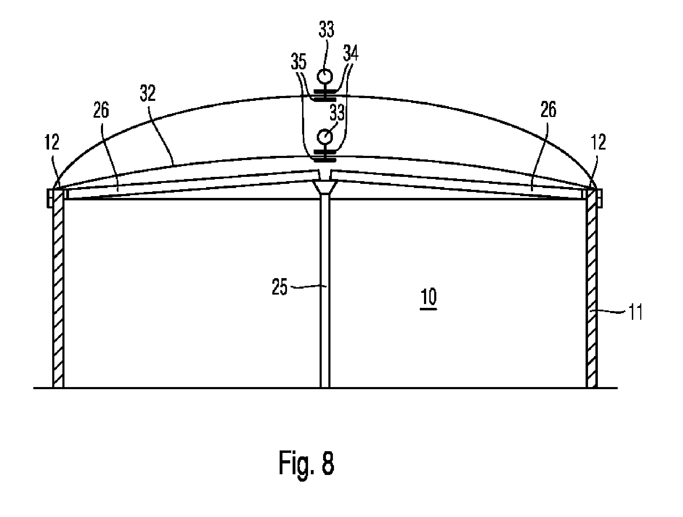

Fig. 8 zeigt einen bereits fertig aufgebauten Biogasfermenter 10. Der Innenraum des Fermenters 10 ist bereits mit einem Dach aus einer Tragluftfolie 31 und einer darunter angeordneten Membranfolie 32 abgedeckt. Der Raum unterhalb der Membranfolie 32 bildet einen Speicher für das Biogas. Die Tragluftfolie 31 und die Membranfolie 32 werden in an sich bekannter Weise auf die Balken 26 aufgelegt und außen am oberen Rand 12 der Wandung 11 des Biogasfermenters 10 befestigt. Wie in Fig. 8 erkennbar, ist etwa mittig in der Tragluftfolie 31 und in der Membranfolie 32 ein Befestigungsmittel 33, konkret eine Öse, für eine Sicherheitsleine angebracht. Das Befestigungsmittel 33 weist oberhalb der jeweiligen Folie 31, 32 einen Kragen 34 auf, der außen an der Folie 31, 32 anliegt. Auf der Unterseite der Folie 31, 32 ist korrespondierend eine Gegenplatte 35 angeordnet. Diese ist mit dem Kragen 34 verschraubt, so daß auf diese Weise das Befestigungsmittel 33 an der Folie 31, 32 befestigt ist.

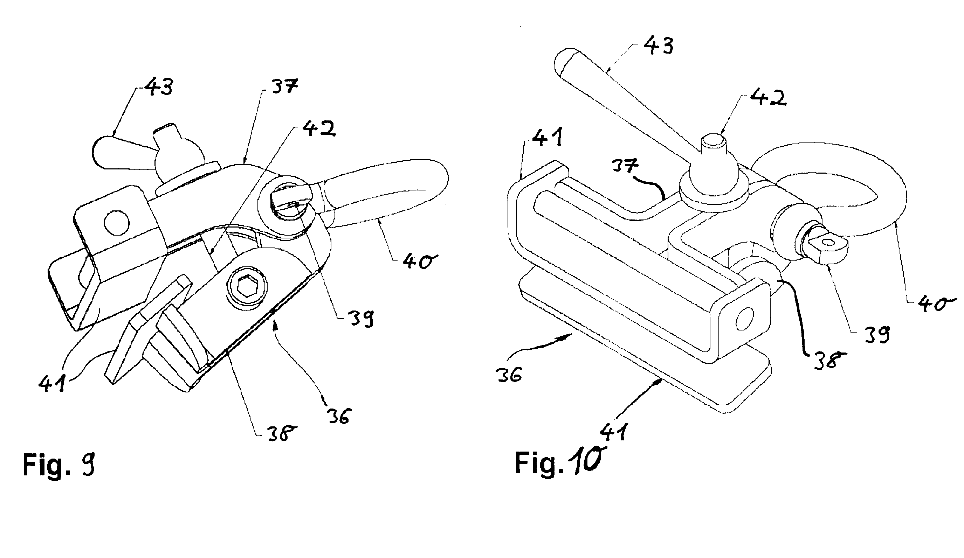

Die Fig. 9 zeigt eine alternative Ausführungsform des Befestigungsmittel 33, welche auch unabhängig von der vorliegenden Erfindung denkbar ist. Das Befestigungsmittel 33 weist ein Befestigungsorgan 36 auf, welches seinerseits einen oberen Zangenarm 37 und einen unteren Zangenarm 38 aufweist, welche durch ein Gelenk gelenkig miteinander verbunden sind. Dieses Gelenk ist ein Sperrbolzen 39 eines Schäkels 40. Über diesen Sperrbolzen 39 wird der Schäkel 40 gleichzeitig mit dem Befestigungsorgan 36, also dem oberen Zangenarm 37 und dem unteren Zangenarm 38, verbunden.

Die Zangenarme 37, 38 weisen an ihren dem Sperrbolzen 39 gegenüberliegenden Enden Klemmstücke 41 auf, zwischen denen eine Folie eines Foliendaches eingeklemmt werden kann. Hierzu wird das Befestigungsorgan 36 mittels einer Spannschraube 42 in Schließrichtung der Klemmstücke 41 vorgespannt. Zum Betätigen der Spannschraube 42 dient ein Handknebel 43.

Sobald eine Folie für ein Foliendach, beispielsweise eines Biogasfermenters, grob auf demselben ausgebreitet ist, wird das insoweit beschriebene Befestigungsmittel 33 etwa mittig an der Folie befestigt. Hierzu wird ein Abschnitt der Folie ergriffen und zwischen den Klemmstücken 41 eingeführt. Sodann werden die Zangenarme 37, 38 mittels der Spannschraube 42 durch Betätigen des Handknebels 43 aufeinander zu bewegt und so das Befestigungsorgan 36 geschlossen. Dabei klemmt das Befestigungsorgan den entsprechenden Abschnitt der Folie zwischen den Klemmstücken 41 ein. Das Befestigungsmittel 33 ist nun insoweit sicher an dem Foliendach befestigt und Sorgleinen können an dem Schäkel 40 eingehängt werden. Kommt es nun zum Zug auf die Sorgleine, wird das Gelenk 39 von den Klemmstücken 41 weggezogen. Aufgrund der besonderen Konstruktion des Befestigungsorgans 36 werden die Klemmstücke 41 dadurch noch weiter aufeinander zu gedrückt und die Haltekraft verstärkt, so daß auch bei versehentlich nicht vollständig geschlossener Spannschraube 42 noch eine ausreichende Haltekraft gewährleistet ist.

### Bezugszeichenliste:

- 10: Biogasfermenter
- 11: Wand
- 12: Rand
- 13: Schiene
- 14: Fuß
- 15: Steg
- 16: Sicherungseinrichtung
- 17: Sicherungsgurt
- 18: Person
- 19: Arbeitsgerüst
- 20: Rolle
- 21: Sicherungseinrichtung
- 22: Sicherungsgurt
- 23: Leiter
- 24: Sicherungseinrichtung
- 25: Stütze
- 26: Balken
- 27: Sicherungseinrichtung
- 28: Stab
- 29: Laufleine
- 30: Sicherungsgurt
- 31: Tragluftfolie
- 32: Membranfolie
- 33: Befestigungsmittel
- 34: Kragen
- 35: Gegenplatte
- 36: Befestigungsorgan
- 37: Zangenarm
- 38: Zangenarm
- 39: Sperrbolzen
- 40: Schäkel
- 41: Klemmstück
- 42: Spannschraube
- 43: Handknebel

## Patentansprüche

1. Behälterartiges Bauwerk, insbesondere einen Gärbehälter für eine Biogasanlage, mit einer umlaufenden Wand (11), welche einen nach oben offenen Innenraum umschließt, **dadurch gekennzeichnet , daß** der Innenraum mittels einer Folie (31, 32) abgedeckt ist, wobei an der Folie (31, 32) ein Befestigungsmittel (33) für eine Sicherheitseinrichtung angeordnet ist.

2. Bauwerk nach Anspruch 1, **dadurch gekennzeichnet, daß** das Befestigungsmittel (33) etwa mittig an der Folie (31, 32) angeordnet ist.

3. Bauwerk nach einem der vorhergehenden Ansprüche, **dadurch gekennzeichnet, daß** im Bereich des oberen Randes (12) der Wand (11) eine umlaufende Schiene (13) oder ein Seil angeordnet ist, wobei vorzugsweise die Schiene (13) im Querschnitt L-, C-, U- oder T-Förmig oder als Flachstahl ausgebildet ist mit wenigstens einem etwa parallel zur Wand verlaufenden Steg (15).

4. Bauwerk nach Anspruch 3, **dadurch gekennzeichnet, daß** die Schiene (13) zwei keilförmig aufeinander zu verlaufende Füße (14) aufweist, an deren von der Wand (11) abgewandten Ende der Steg (15) angebracht ist.

5. Bauwerk nach einem der Ansprüche 3 bis 4, **dadurch gekennzeichnet, daß** an der Schiene (13) eine Sicherungseinrichtung (16, 21, 24, 27) und/oder daß an der Schiene (13) ein Arbeitsgerüst (19) angeordnet ist.

6. Bauwerk nach Anspruch 5, **dadurch gekennzeichnet, daß** eine weitere Sicherungseinrichtung, insbesondere ein aufrechter, etwa mittig im Bauwerk angeordneter Stab (28), vorgesehen ist, welcher mit der zweiten Sicherungseinrichtung (16, 21, 24, 27) durch eine Laufleine (29) oder eine Stange verbunden ist.

7. Bauwerk nach Anspruch 5, **dadurch gekennzeichnet, daß** zwei einander etwa diametral gegenüberliegende Sicherungseinrichtungen (27) an der Schiene (13) angeordnet sind, welche durch eine Laufleine (29) oder eine Stange verbunden sind.

8. Bauwerk nach einem der vorhergehenden Ansprüche, **dadurch gekennzeichnet, daß** das Befestigungsmittel (33) ein Befestigungsorgan (36) und eine Öse (40) für die Sicherheitseinrichtung aufweist und daß das Befestigungsorgan (36) zangenartig ausgebildet und an dem Foliendach festklemmbar ist.

9. Bauwerk nach Anspruch 8, **dadurch gekennzeichnet, daß** ein Abschnitt der Öse (40) gleichzeitig ein Gelenk (39) des zangenartigen Befestigungsorgans (36) ist und/oder daß die Öse ein Schäkel (40) und ein Sperrbolzen (39) des Schäkels (40) das Gelenk ist.

10. Bauwerk nach einem der Ansprüche 8 oder 9, **dadurch gekennzeichnet, daß** das zangenartige Befestigungsorgan (36) mittels einer Spannschraube (42) feststellbar ist.
